Europäisches Patentamt

(19) European Patent Office

Office européen des brevets

(11) Veröffentlichungsnummer: **0 038 935**
**B1**

(12) **EUROPÄISCHE PATENTSCHRIFT**

(45) Veröffentlichungstag der Patentschrift:
12.12.84

(51) Int. Cl.³: **G 01 N 33/86**, G 01 N 33/54

(21) Anmeldenummer: 81102274.8

(22) Anmeldetag: 26.03.81

(54) Verfahren zur immunologischen Bestimmung von Enzymen, Mittel zur Durchführung des Verfahrens und seine Verwendung.

(30) Priorität: 30.04.80 DE 3016575

(43) Veröffentlichungstag der Anmeldung:
04.11.81 Patentblatt 81/44

(45) Bekanntmachung des Hinweises auf die Patenterteilung:
12.12.84 Patentblatt 84/50

(84) Benannte Vertragsstaaten:
BE CH DE FR GB IT LI NL SE

(56) Entgegenhaltungen:
DE - A - 2 641 840
DE - A - 2 708 985
DE - B - 2 206 103
FR - A - 2 357 899

CHEMICAL ABSTRACTS, Band 90, Nr. 19, 7. Mai 1979,
Seite 203, Nr. 14744m Columbus, Ohio, U.S.A. E.F. PLOW
et al.: "Immunochemical characterization of the
plasmin-antiplasmin system. Basis for the specific
detection of the plasmin-antiplasmin complex by latex
agglutination assays"
CHEMICAL ABSTRACTS, Band 83, Nr. 15, 13. Oktober
1975, Seite 244, Nr. 128434a Columbus, Ohio, U.S.A. D.
COLLEN et al.: "Tanned red cell hemagglutination
inhibition immunoassay for the quantitative estimation
of thrombin-antithrombin III and

(73) Patentinhaber: Merck Patent Gesellschaft mit
beschränkter Haftung, Frankfurter Strasse 250,
D-6100 Darmstadt (DE)

(72) Erfinder: Neumann, Siegfried, Dr., Battenbergstrasse 11,
D-6104 Seeheim-Jugenheim 2 (DE)
Erfinder: Gunzer, Gerhard, Dr.,
Albert-Schweizer-Strasse 15, D-6107-Reinheim 5 (DE)
Erfinder: Hennrich, Norbert, Dr., Haydnweg 14,
D-6100 Darmstadt (DE)
Erfinder: Lang, Hermann, Dr., Ahastrasse 7,
D-6100 Darmstadt (DE)

(56) Entgegenhaltungen: (Fortsetzung)
plasmin-alpha1-antiplasmin complexes in human
plasma"
CLINICAL CHEMISTRY, Band 26, Nr. 10, September
1980, Seiten 1380-1391 Easton, Pennsylvania, U.S.A.
JAWED FAREED et al.: "New perspectives in
coagulation testing"
W. Vogt, Enzymimmunoassay, 1978, Seiten 7,8

## Beschreibung

Die Erfindung betrifft Verfahren und Mittel zur immunologischen Bestimmung von Enzymen, die mit einem Inhibitor einen Enzym-Inhibitor-Komplex bilden, insbesondere zur Bestimmung von Proteasen.

In entzündlichen Prozessen spielen Proteasen aus polymorphkernigen Leukocyten und Makrophagen eine kausale Rolle bei der Schädigung der betroffenen Gewebe. Die leukocytären Proteasen wie z. B. Elastase oder Collagenase sind in den Lysosomen der weißen Blutzellen lokalisiert und werden nach pathologischen Reizen in das extrazelluläre Milieu abgegeben. Sie bewirken dort einen Abbau von Bindegewebssubstanzen und nach Eintritt in den Blutkreislauf eine Störung der Homeostase, z. B. durch Abbau von Gerinnungsfaktoren und Fibrinogen. Der quantitative Nachweis von leukocytären Proteasen im Blut ist deshalb sehr wertvoll für die Beurteilung der Akuität von Entzündungen, für die Verlaufsbeobachtung bei chronischen Entzündungen, für die Kontrolle der Therapie und die Prognostik. Die Bestimmung dieser Faktoren erscheint immer dann nötig, wenn im Zusammenhang mit Erkrankungen Leukocytosen auftreten, wie bei bestimmten Infektionskrankheiten, Intoxikationen, Gewebsnekrosen, endokrinologischen Erkrankungen, bei malignen Tumoren usw.

Der exakte quantitative Nachweis von Leukocytenproteasen im Blut wurde bisher dadurch behindert, daß diese Proteine beim Eintritt in die Zirkulation rasch Komplexe mit den im Blut reichlich vorhandenen Proteaseinhibitoren, z. B. mit $\alpha_1$-Antitrypsin, $\alpha_2$-Makroglobulin und anderen, bilden. Die Komplexe mit $\alpha_1$-Antitrypsin sind stöchiometrisch aufgebaute Verbindungen aus einem Molekül Protease und einem Molekül Inhibitor unter Bildung einer kovalenten Bindung. Im Komplex ist die Protease enzymatisch inaktiv. Daher ist die Bestimmung der enzymatischen Aktivität völlig ungeeignet für die quantitative Erfassung der Protease-Konzentrationen im Plasma, Serum oder auch in anderen Körperflüssigkeiten wie Synovialflüssigkeit, Exsudat, Gewebsextrakt usw.

Unter den leukocytären Proteasen hat die Elastase (EC 3.4.21.11) eine besondere Bedeutung: Sie kommt in den Leukocyten reichlich vor und stellt zusammen mit Collagenase 5% des Trockengewichts von Granulocyten. Das Enzym hat eine geringe Substratspezifität und kann viele verschiedene, biologisch wichtige Substanzen proteolytisch abbauen, wie z. B. Elastin, Proteoglycane, Collagen, IgC, Complementfaktoren, Fibrinogen und Gerinnungsfaktoren. Elastase liegt im Plasma zu 92% gebunden an $\alpha_1$-Antitrypsin und zu 8% gebunden an andere Inhibitormoleküle vor. Komplexe aus Elastase und $\alpha_1$-Antitrypsin wurden bei entzündlichen Prozessen z. B. in Plasma, Serum, Peritonealflüssigkeit und Eiter nachgewiesen.

Da die Proteasen in Körperflüssigkeiten stets gebunden an Inhibitoren vorkommen, ist deren Nachweis praktisch nur mit Hilfe immunologischer Methoden möglich. Bekannte Methoden sind die Elektroimmunodiffusion, der Radioimmunoassay und die Agglutinationstests bzw. Agglutinationshemmtests. Diese Methoden haben jedoch erhebliche Nachteile: Die Elektroimmunodiffusion ist langwierig und für Routinebestimmungen ungeeignet; die Auswertung ist nur qualitativ und schlecht reproduzierbar. Der Radioimmunoassay ist ebenfalls zeitaufwendig und nachteilig wegen der Verwendung radioaktiver Substanzen. Besondere Nachteile sind: Notwendigkeit spezieller Laborausrüstungen, besondere Sicherheitsmaßnahmen wegen der Strahlungsgefahr, geringe Stabilität der radioaktiv markierten Reagenzien, Entsorgung usw.

Aus der DOS 2 641 840 ist ein Agglutinationstest bekannt, bei dem das zu untersuchende Plasma mit einer Suspension von Teilchen aus synthetischem Harz oder Blutkörperchen, die Antikörper gegen einen Enzym-Inhibitor-Komplex auf ihrer Oberfläche tragen, kontaktiert und dann unmittelbar beobachtet wird, ob Agglutination vorkommt oder nicht. Diese Methode hat den Nachteil, daß sie nur semiquantitative Ergebnisse liefert und nicht routinefähig ist. Die verwendeten Antikörper müssen durch aufwendige Herstellungsverfahren spezifisch gemacht werden für antigene Determinanten, die nur in den Enzym-Inhibitor-Komplexen vorhanden sind.

In der DAS 2 206 103 ist eine Methode beschrieben, bei der eine zu bestimmende Komponente mit einem unlöslich gemachten Bindungspartner reagiert und einer dieser beiden Partner über ein enzymmarkiertes Indikatormolekül nachgewiesen wird. Die spezifische quantitative Bestimmung eines Enzym-Inhibitor-Komplexes ist nach dieser Methode nicht möglich.

Der vorliegenden Erfindung liegt die Aufgabe zugrunde, Verfahren und Mittel zur quantitativen Bestimmung von Enzymen, die in Form von Enzym-Inhibitor-Komplexen vorliegen, zur Verfügung zu stellen, mit denen die geschilderten Nachteile der bekannten Methoden vermieden werden können.

Erfindungsgemäß wurde die Aufgabe dadurch gelöst, daß man einen Enzym-Inhibitor-Komplex mit Hilfe eines Festphasen-Antikörpers gegen den Enzymanteil bindet und die Bestimmung über einen markierten Antikörper gegen den Inhibitoranteil des Molekülverbandes erfolgt.

Gegenstand der Erfindung ist ein Verfahren zur Bestimmung von Enzymen mit Hilfe von an einen wasserunlöslichen Träger gebundenen Antikörpern gegen das zu bestimmende Enzym, das dadurch gekennzeichnet ist, daß man Enzyme, die mit einem Inhibitor einen Enzym-Inhibitor-Komplex bilden, in Form eines solchen Komplexes mit den gebundenen Antikörpern inkubiert, die Probelösung entfernt, den ausgewaschenen, mit dem Enzym-Inhibitor-Komplex beladenen Antikörper-Träger mit einem Kupplungsprodukt, gebildet aus einem spezifisch mit dem Inhibitor reagierenden Antikörper und einer Markersubstanz, inkubiert, das ungebundene Kupplungsprodukt entfernt und die Konzentration der Markersubstanz an der festen Phase als Maß für die zu bestimmende Enzymkonzentration ermittelt.

Ferner betrifft die Erfindung Mittel zur Durchführung dieses Verfahrens, enthaltend mit Antikörper gegen das zu bestimmende Enzym beschichtete Reaktionsgefäße und ein Kupplungsprodukt, gebildet aus einem spezifisch mit dem Inhibitor reagierenden Antikörper und einer Markersubstanz. Ein weiterer Gegenstand der Erfindung besteht in der Verwendung dieser Mittel zur Bestimmung von Enzymen, die mit Inhibitoren einen Enzym-Inhibitor-Komplex bilden.

Mit dem erfindungsgemäßen Verfahren und Mittel können prinzipiell alle Enzyme bestimmt werden, die mit einem Inhibitor einen Enzym-Inhibitor-Komplex zu bilden vermögen und deshalb der direkten enzymatischen Aktivitätsbestimmung nicht zugänglich sind. Das Verfahren eignet sich besonders zur Bestimmung von Komplexen aus Proteasen mit den für sie jeweils relevanten Proteaseinhibitoren wie Elastase/$\alpha_1$-Antitrypsin, Thrombin/Antithrombin III, Plasmin/Antiplasmin, Trypsin/$\alpha_1$-Antitrypsin usw. Das Verfahren ist spezifisch, einfach, schnell und empfindlich. Für die Durchführung werden lediglich drei spezifisch hergestellte Reagenzien benötigt (Antikörper-beschichtete Reaktionsgefäße, enzymmarkierte Antikörper, Lösung mit definierter Konzentration des Enzym-Inhibitor-Komplexes), die Ausführung ist mit der apparativen Ausstattung eines normalen klinisch-chemischen Labors möglich. Das Verfahren beruht auf der spezifischen Bindung des Enzym-Inhibitor-Komplexes an immobilisierten Antikörpern gegen das Enzym und einer nachfolgenden quantitativen Bestimmung des gebundenen Komplexes mit markierten Antikörpern gegen den Inhibitor. Zum Nachweis werden also zwei Antiseren mit absolut unterschiedlicher Bindungsspezifität, nämlich spezifisch für einen der beiden Partner des Molekülverbandes, eingesetzt.

Ein für das z. B. im Protease-Proteaseinhibitor-Komplex vorliegende proteolytische Enzym spezifischer Reaktant wird an einen festen Träger gebunden. Reaktanten spezifisch für die Protease sind Antikörper oder deren Antigen-bindende Fragmente. Spezifische Antikörperpräparationen werden aus den Seren spezifisch immunisierter Tiere isoliert. Als Immunogen wird die Protease in gehemmtem Zustand nach Reaktion mit niedermolekularen Hemmstoffen oder auch in unbehandeltem Zustand verwendet. Geeignete Versuchstiere für die Herstellung der Antiseren sind beispielsweise Schafe oder Kaninchen, jedoch sind auch andere Versuchstiere geeignet.

Der in fester Phase vorliegende Antikörper gegen die Protease kann trägerfrei vorliegen, z. B. durch Vernetzung mit polyfunktionellen Reagentien wie Dialdehyden, Dioxiden usw. unlöslich gemacht werden, vorzugsweise ist der Antikörper aber an ein festes Trägermaterial gebunden. Der feste Träger kann aus jedem gegenüber den im Test verwendeten Substanzen inerten Material bestehen, an das Antikörper gegen Proteasen gebunden werden können. Geeignete Träger sind z. B. Formkörper wie amorphe Partikel, Kugeln, Plättchen, Folien oder Reagenzgefäße aus anorganischem Material (z. B. Glas) oder organischem Material (z. B. Homo- oder Copolymere von Vinylverbindungen wie Olefinen, Vinylacetat, Vinylchlorid, Vinylidenchlorid, Tetrafluoräthylen, Styrol, Acrylsäure oder Methacrylsäure, ferner Polymere von Formaldehyd und cyclischen Acetalen sowie Polykondensate wie Polyester, Polycarbonate oder Polyamide oder vernetzte Kohlenhydrate und vernetzte Polypeptide), weiterhin biologische Partikel wie Zellen (z. B. Erythrocyten).

Die Bindung der Antikörper an den festen Träger kann wahlweise adsorptiv, adhäsiv oder kovalent erfolgen. Es ist möglich, die Antikörper nach den üblichen Methoden der Proteinchemie zur kovalenten Bindung von biologisch aktiven Polypeptiden an festen Trägern zu fixieren. Typische Beispiele hierfür sind die Aktivierung von Trägeroberflächen nach der Bromcyan-Methode oder die oberflächliche Vernetzung der Antikörper auf dem Träger durch polyfunktionelle Vernetzungsmittel wie Glutardialdehyd und dergleichen.

Es wurde gefunden, daß bei zahlreichen Trägermaterialien die Antikörper gegen die Protease durch Adsorption oder Adhäsion ausreichend fest an die Oberfläche des Trägers gebunden werden können. Für die Beschichtung wird der Träger mit den Antikörpern oder ihren Antigenbindenden Fragmenten in Kontakt gebracht. Beispielsweise wird das Protein in einer wäßrigen Lösung, vorzugsweise einer 0,15-M-Kochsalzlösung oder phosphatgepufferter isotonischer Kochsalzlösung (=PBS) mit pH 7,2, in einer Proteinkonzentration von 1 μg bis 1 g/l, vorzugsweise 10 mg/l, gelöst. Die Proteinlösung wird durch Verdünnen des Blutplasmas oder Blutserums spezifisch immunisierter Tiere hergestellt, vorzugsweise aber durch Lösung von gereinigtem Immunoglobulin aus dem Antiserum. Die Lösung wird 1 min bis 5 Tage, vorzugsweise 24 Stunden lang, mit dem Träger stehengelassen. Danach wird die Lösung vom Träger getrennt, beispielsweise durch Ausgießen aus dem Behälter; der Träger wird mit Puffer, der zweckmäßigerweise ein Detergens enthält (z. B. PBS pH 7,2 mit 0,05% Polyoxyäthylensorbitanmonolaurat), gewaschen.

Die Bestimmung der Protease-Proteaseinhibitor-Komplexe erfolgt in der Weise, daß der Träger, der mit spezifischen Antikörpern gegen die zu bestimmende Protease oder mit den Antigenbindenden Fragmenten aus den Antikörpern beschichtet wurde, 1 min bis 48 Stunden, vorzugsweise 2 Stunden, mit der die Protease-Proteaseinhibitor-Komplexe enthaltenden Flüssigkeit, z. B. Serumprobe, inkubiert wird. In parallelen Ansätzen werden Proben mit bekannter Konzentration der Protease-Proteaseinhibitor-Komplexe eingesetzt. Diese Proben werden durch Verdünnen eines Reaktionsgemisches aus hochgereinigter Protease und hochgereinigtem Proteaseinhibitor in Puffer (z. B. PBS pH 7,2) oder in Humanplasma, welches vorher 30 min bei 56° C inaktiviert wurde, zubereitet. Nach Beendigung der Inkubation wird die Lösung vom Träger abgetrennt, der Träger wird zweckmäßigerweise zur Entfernung überschüssiger Probenbestandteile mit Puffer (z. B. PBS pH 7,2) gewaschen. Anschließend wird der so behandelte Träger mit einer Lösung eines markierten zweiten Reaktanten, der spezifisch für den komplexgebundenen Proteaseinhibitor ist, inkubiert. Die Dauer hängt in gewissem Umfang von den Bedingungen hinsichtlich pH-Wert, Temperatur und Konzentrationen ab. Im allgemeinen reicht eine Einwirkungszeit von 2 bis 24 Stunden. Reaktanten für diesen Versuchsschritt sind Antikörper oder Antikörperfragmente, die gegen antigene Determinanten des komplexgebundenen Proteaseinhibitormoleküls gerichtet sind. Spezifische Antikörperpräparationen werden aus den Seren spezifisch immunisierter Tiere isoliert. Es hat sich als zweckmäßig erwiesen, die Antikörperfraktion aus diesen Antiseren durch Verfahren der Immunadsorption an immobilisiertem Antigen, Antigen-spezifisch anzureichern und von Antikörpern unbekannter Spezifität zu trennen.

Als Markierungsmittel für den zweiten Reaktanten werden z. B. Enzyme, Lumineszenz- oder Elektronendichtemarker, fluoreszierende oder radioaktive Substanzen verwendet. Bevorzugt werden im Rahmen der Erfindung solche Markierungsenzyme verwendet, deren Aktivität sich leicht durch einen optischen Test bestimmen läßt, insbesondere durch eine Farbreaktion im sichtbaren Licht oder durch eine Änderung der Konzentration von NADH oder NADPH. Typische Beispiele für geeignete Markierungsenzyme sind alkalische Phosphatase, Peroxidase, Glucoseoxidase, $\alpha$- und $\beta$-Galactosidase, Glucoamylase, Invertase, Malatdehydrogenase oder Glucose-6-phosphat-dehydrogenase. Kovalente Bindungen der jeweiligen Markierungsenzyme mit den spezifischen Antikörpern gegen Proteaseinhibitoren oder Antigen-bindenden Antikörperfragmenten werden nach literaturbekannten Verfahren durchgeführt [z. B. J. Histochem. Cytochem. 22, 1084 (1974); Bull. Soc. Chim. Biol. 50, 1169 (1968)].

Die im Test einzusetzenden optimalen Verdünnungen der markierten Antikörper gegen den komplexgebundenen Proteaseinhibitor werden durch Vorversuche ermittelt. Sie dürfen keine unspezifische, d. h. ohne Anwesenheit von träger-gebundenem Protease-Proteaseinhibitor-Komplex nachweisbare Verbindung mit dem Träger eingehen. Zu diesem Zweck wird der Lösung des markierten Antikörpers ein Detergens, z. B. 1 mM Natriumdodecylsulfat, zugesetzt. Nach Beendigung der Inkubation wird die Lösung vom Träger getrennt, beispielsweise durch Abgießen aus dem Behälter; der Träger wird mehrmals mit Puffer, welcher zweckmäßigerweise ein Detergens enthält, gewaschen und anschließend wird die Menge des am Träger gebundenen Markierungsmittels mit üblichen Methoden bestimmt. Zur Kontrolle auf unspezifische Bindung von Markierungsmittel am Träger dienen Versuche, die, wie oben beschrieben, jedoch mit Proben ohne Protease-Proteaseinhibitor-Komplex der zu bestimmenden Zusammensetzung durchgeführt werden.

Der Gehalt an Protease-Proteaseinhibitor-Komplex mit der zu bestimmenden Protease in einer biologischen Probe wird durch Vergleich mit den oben beschriebenen Standardproben quantitativ bestimmt.

Die erfindungsgemäßen Mittel können zusätzlich noch übliche Stabilisierungsmittel wie Proteinadditive (Rinderserumalbumin oder Ovalbumin), Kohlenhydrate, Glycerin, Bateriostatika, Fungizide usw. enthalten.

Die Erfindung wird durch die folgenden Beispiele näher erläutert.

Beispiel 1

Quantitativer immunologischer Nachweis von Komplexen aus Leukocyten-Elastase und $\alpha_1$-Antitrypsin

a) Herstellung von Antiserum gegen Leukocyten-Elastase

Elastase wurde aus menschlichen polymorphkernigen Leukocyten gewonnen und durch Affinitätschromatographie und Ionenaustauschchromatographie so weit gereinigt, daß in der Elektrophorese auf Polyacrylamidgel bei pH 4,3 und in der Immunelektrophorese mit Antiserum gegen Humanserumproteine kein verunreinigendes anderes Protein mehr nachweisbar war. Das gefriergetrocknete, salzfreie Protein wurde in 0,15 M Kochsalzlösung mit einer Konzentration von 2 mg/ml gelöst. 1 ml der Lösung wurde mit 1 ml kompletten Freundschen Adjuvans emulgiert. Diese Emulsion wurde Schafen oder Ziegen an 3 bis 4 Stellen i. m. in die Hinterläufe injiziert. Gleiche Injektionen erfolgten noch zweimal im Abstand von je drei Wochen. 21 Tage nach der letzten Injektion wurde Blut entnommen. Weitere Injektionen erfolgten im Abstand von 12 Wochen nach der letzten Blutentnahme. Jeweils 14 Tage nach der Boosterinjektion wurde Blut entnommen. Das Serum wurde nach üblichen Verfahren aus dem Blut gewonnen, durch Zusatz von 0,05% Natriumazid sowie 0,05% Natriumäthylquecksilberthiosalicylat stabilisiert und bei −20° C gelagert.

b) Herstellung von Antiserum gegen $\alpha_1$-Antitrypsin

Als Antigen wird hochgereinigtes $\alpha_1$-Antitrypsin aus Humanplasma verwendet. Antiserum gegen $\alpha_1$-Antitrypsin wurde in Schafen oder Ziegen nach dem gleichen Impfschema wie in a) beschrieben hergestellt. Für die Immunisation von Kaninchen wurde 1 mg gefriergetrocknetes, salzfreies $\alpha_1$-Antitrypsin in. 0,5 ml 0,15 M Kochsalzlösung gelöst. Die Lösung wurde mit 0,5 ml kompletten Freundschen Adjuvans emulgiert, und die Emulsion wurde dem Tier an 2 bis 3 Stellen i. m. in die Hinterläufe injiziert. Eine gleiche Emulsion erfolgte 4 Wochen später s. c. in den Nacken. 7 Tage danach wurden ca. 40 ml Blut aus der marginalen Ohrvene entnommen. 4 Wochen später erfolgte jeweils eine weitere Boosterinjektion s. c. und im Abstand von 7 Tagen darauf eine Blutentnahme. Das Serum wurde, wie in a) beschrieben, behandelt.

### c) Isolierung von Immunglobulin aus den Antiseren

Die IgG-Fraktion aus den Antiseren gegen Elastase bzw. gegen $\alpha_1$-Antitrypsin wurde nach üblichen Verfahren [Scand. J. Immunol. 2, Suppl. 1, 161 (1973) oder Arch. Biochim. Biophys. 134, 279 (1969)] aus den Seren isoliert und in salzfreier, gefriergetrockneter Form gelagert.

### d) Reinigung von Antikörpern gegen $\alpha_1$-Antitrypsin durch Immunadsorption

Antikörper gegen den Proteaseinhibitor wurden durch Chromatographie von gereinigten Immunglobulinen (nach c)) aus Antiseren gegen $\alpha_1$-Antitrypsin (nach b)) über Sepharose 4B oder über substituierte Agarose, welche kovalent gebundenes $\alpha_1$-Antitrypsin enthielten, immunspezifisch isoliert. Antikörper gegen den Proteaseinhibitor blieben an dem immobilisierten Antigen haften und wurden mit 0,1 M Glycin/HCl-Puffer pH 2,8 oder 3 M Kaliumrhodanidlösung eluiert. Cirka 5 bis 20% des aufgegebenen Immunoglobulins wurden auf diese Weise immunspezifisch isoliert; Immunoglobuline mit anderer Spezifität wurden somit entfernt. Das Eluat mit den spezifischen Antikörpern wurde gegen Phosphatgepufferte Saline und danach gegen 0,01 M Ammoniumbicarbonatlösung dialysiert. Anschließend wurde das Protein gefriergetrocknet.

### e) Herstellung von Enzym-Antikörper-Konjugaten

Immunspezifisch gereinigte Antikörper (nach d)) wurden mit alkalischer Phosphatase aus Kälberdünndarm unter Verwendung von 0,2% Glutardialdehyd vernetzt [Biochim. Biophys. Acta 251, 427 (1971)]. Das Reaktionsprodukt wurde über eine Säule mit Bio-Gel A 1,5 m (26 mm × 900 mm) chromatographiert. Die Enzym-Antikörper-Komplexe waren im ersten Maximum der AP-Aktivität des Eluats enthalten. Die Lösung wurde durch Zugabe von Ovalbumin (ad 0,1%) und Natriumazid (ad 0,05%) stabilisiert und bei 4° C gelagert.

### f) Beschichtung von Reaktionsgefäßen mit Antikörpern gegen Elastase

Antikörper gegen Elastase, hergestellt nach a) und aufgereinigt nach c) wurde durch Adsorption an Kunststoff-Reaktionsgefäße immobilisiert. Als Reaktionsgefäße wurden Reagenzröhrchen aus Polystyrol 12 × 55 mm, Rundküvetten oder Platten für die Mikrotitration verwendet. Im typischen Ansatz wurden die Röhrchen oder Küvetten mit 1 ml einer IgG-Lösung mit 10 µg IgG/ml (in 0,15 M Kochsalzlösung mit 0,02% Natriumazid) gefüllt. Für die Platten wurden pro Depot 0,2 ml einer IgG-Lösung mit 50 µg/ml eingesetzt. Die Gefäße wurden verschlossen und über Nacht bei 4° C stehengelassen. Vor dem Gebrauch im Test wurde die Flüssigkeit dekantiert oder abgesaugt. Die Gefäße wurden dann mehrere Male mit Spüllösung (0,15 M Kochsalzlösung mit 0,05% eines Detergens und 0,02% Natriumazid) gewaschen und kurz an der Luft getrocknet.

### g) Herstellung von Komplexen aus Leukocyten-Elastase und $\alpha_1$-Antitrypsin

Als analytische Probe für den quantitativen Nachweis wurde der Komplex aus Protease und Proteaseinhibitor in vitro durch Inkubation der beiden isolierten hochgereinigten Komponenten erzeugt. Hierzu wurden 0,2 ml einer Lösung von 0,5 mg reiner Leukocyten-Elastase (human) in 1 ml (gelöst in 10 mM Tris/HCl-Puffer pH 7,4 mit 0,12 M Natriumchlorid) und 0,2 ml einer Lösung von 2,8 mg reinem $\alpha_1$-Antitrypsin (aus Humanplasma) in 1 ml (gelöst in 50 mM Tris/HCl-Puffer pH 8,8 mit 0,05 M Natriumchlorid) gemischt und danach 2 Stunden bei 37° C inkubiert. Das Gemisch wurde anschließend bei −20° C gelagert.

Die Analyse mit der Immunelektrophorese zeigte, daß die gesamte Elastase im Inkubationsgemisch zu dem Elastase/$\alpha_1$-Antitrypsin-Komplex umgesetzt wurde.

### h) Wiederfindung von Komplexen aus Leukocyten-Elastase und $\alpha_1$-Antitrypsin

In Mikrotiterplatten, die nach f) mit Antikörpern gegen Elastase beschichtet und für den Gebrauch vorbereitet wurden, wurden 0,2 ml Probe mit einem Gehalt von 0,1−10 ng Elastase im Inhibitorkomplex pro Depot eingefüllt. Die Proben mit dem definierten Elastase-Gehalt wurden durch Verdünnen des Enzym-Inhibitor-Gemisches nach g) erhalten; als Verdünnungsmittel diente Phosphat-gepufferte Saline. Zur Bestimmung von Blindwerten wurden einige Depots parallel mit 0,2 ml Verdünnungsmittel ohne Enzym-Inhibitor-Komplex gefüllt. Nach einer Inkubation von 2 Stunden bei Raumtemperatur wurde der Inhalt der Depots abgesaugt. Nach dreimaligem Waschen mit Phosphat-gepufferter Saline wurde Lösung mit Enzym-Antikörper-Konjugat nach e) (0,2 ml pro Depot) eingefüllt. Die Lösung wurde vorher auf ca. 800 U/l AP-Aktivität eingestellt. Verdünnungsmittel hierfür war 10 mM Tris/HCl-Puffer pH 7,5 mit 2 mM Magnesiumchlorid, 0,025 mM Zinkchlorid, 1% Ovalbumin, 1 mM Natriumdodecylsulfat und 0,02% Natriumazid. Die Platten wurden luftdicht verschlossen und bei Raumtemperatur über Nacht inkubiert. Danach wurde die Flüssigkeit abgesaugt. Die Platten wurden dreimal gespült und kurz an der Luft getrocknet. Danach wurde Puffer-Substrat-Lösung für die Bestimmung der Aktivität der alkalischen Phosphatase mit 0,2 ml pro Depot in zeitversetzter Reihe eingefüllt. Nach 10 min Inkubation bei Raumtemperatur wurde das Reaktionsgemisch aus den Depots in eine Mikroküvette übergeführt und die Extinktion gemessen. Als Nullwert diente Puffer-Substrat-Lösung. Depots ohne Proteinzusatz ergaben eine Extinktion, die ein Maß für die unspezifische Adsorption der Enzym-Antikörper-Konjugate an die Depots darstellt. In Depots, die Protease-Proteaseinhibitor-Komplexe enthielten, wurden erhöhte Extinktionswerte gefunden. Zur Auswertung wurde die gefundene Extinktion (minus die Extinktion für unspezifische Adsorption) der eingesetzten Menge Elastase (ng) im Inhibitorkomplex gegenübergestellt. Die nachstehende Tabelle gibt die Endwerte der Extinktion bei 405 nm nach 10 min Reaktionszeit in Abhängigkeit von der Elastasemenge an.

| Elastase [ng] | OD$_{405}$/10 min |
| --- | --- |
| 12,5 | 1,000 |
| 6,25 | 1,030 |
| 3,125 | 0,830 |
| 1,563 | 0,540 |
| 0,781 | 0,315 |
| 0,390 | 0,115 |
| 0,195 | 0,030 |
| 0 = Blindwert | 0 |

Die Daten zeigen, daß die Konzentration von Elastase-$\alpha_1$-Antitrypsin-Komplexen auf diese Weise quantitativ bestimmt werden kann. Es besteht ein streng korrelierter Zusammenhang zwischen der Menge von ca. 0,4 ng bis ca. 6 ng komplexgebundener Elastase in der Probe und der gemessenen Extinktion. Als Puffer-Substrat-Lösung wurde eine Lösung verwendet, die folgende Konzentrationen im Test hatte: 10 mM p-Nitrophenylphosphat, 0,5 mM Magnesiumchlorid, 1,0 mM Diäthanolamin-HCl-Puffer pH 9,8.

Beispiel 2

Quantitativer immunologischer Nachweis von Komplexen aus Thrombin und Antithrombin III

a) Isolierung von Immunglobulin aus den Antiseren

Als Quelle der spezifischen Antikörper gegen Protease und Proteaseinhibitor wurden kommerziell erhältliche Antiseren vom Kaninchen gegen menschliches Prothrombin bzw. gegen Antithrombin III aus Humanplasma verwendet. Die IgG-Fraktion aus diesen Antiseren wurden nach bekannten Verfahren isoliert und in salzfreier gefriergetrockneter Form gelagert.

b) Herstellung von enzymmarkierten Antikörpern gegen menschliches Antitrhombin III

Die nach a) isolierte Immunglobulinfraktion aus Antiserum gegen Antithrombin III vom Menschen wurde mit dem Markerenzym alkalische Phosphatase aus Kälberdünndarm chemisch kovalent verbunden, und die derart hergestellten Konjugate aus IgG und Enzym wurden analog Beispiel 1e) weiterbearbeitet.

c) Beschichtung von Reaktionsgefäßen mit Antikörpern gegen Prothrombin

Die nach a) gereinigte Immunoglobulinfraktion aus dem Antiserum gegen menschliches Prothrombin wurde durch Adsorption an Kunststoffgefäßen immobilisiert. Hierfür wurden Mikrotiterplatten aus Polystyrol verwendet, die pro Depot mit 0,2 ml einer Lösung von 50 µg Immunoglobulin pro ml (in 0,15 M Kochsalzlösung mit 0,02% Natriumazid) gefüllt wurden. Die Platten wurden luftdicht eingedeckt und über Nacht bei 4°C stehengelassen. Vor dem Gebrauch im Test wurde die IgG-haltige Flüssigkeit entfernt. Die Gefäße wurden mehrmals mit 0,15 M Kochsalzlösung, welche zusätzlich noch 0,05% Detergens und 0,02% Natriumazid enthielt, gewaschen und kurz an der Luft getrocknet.

d) Herstellung von Molekülkomplexen aus menschlichem Thrombin und menschlichem Antithrombin III

Die Komplexe aus Thrombin und Antithrombin III vom Menschen zur Verwendung als analytische Probe im Test wurden durch Inkubation von Gemischen der beiden Komponenten in hochgereinigter Form erzeugt. Zu diesem Zweck wurden 50 Einheiten Antithrombin III (= Heparin-Cofaktor) und 50 Einheiten Heparin in einem Volumen von 1,26 ml in 10 mM Tris/HCl-Puffer pH 7,5 30 min lang bei 37° C inkubiert, und anschließend wurden dem Gemisch 50 Einheiten Thrombin (entsprechend 16,7 μg Enzymprotein) in 50 μl des gleichen Puffers zugesetzt. Nach einer Inkubation von 2 Stunden bei 37° C wurde die Reaktionslösung und geeignete Verdünnungsstufen in den Test eingesetzt.

### e) Quantitativer Nachweis von Thrombin-Antithrombin III — Komplexen

In die Depots von Mikrotiterplatten, die nach c) mit Antikörpern gegen Prothrombin beschichtet und für den Test vorbereitet wurden, wurden 0,2 ml Probe mit bekanntem Gehalt an Thrombin in komplexgebundener Form eingefüllt. Als Proben mit bekanntem Gehalt wurden Verdünnungsstufen des Inkubationsgemisches nach d) in Phosphat-gepufferter Saline verwendet. Zur Bestimmung von Blindwerten wurden einige Depots mit 0,2 ml Verdünnungsmittel ohne Enzym-Inhibitor-Komplex gefüllt. Nach 2 Stunden Inkubation bei Raumtemperatur wurden alle Depots leergesaugt und dreimal mit Phosphat-gepufferter Saline, welche zusätzlich 0,05% Detergens enthielt, gewaschen. Danach wurden pro Depot 0,2 ml Lösung mit Enzym-markierten Antikörpern gegen Antithrombin III, die nach b) hergestellt wurden, eingefüllt. Die Lösung wurde vorher auf ca. 300 U/l AP-Aktivität eingestellt. Als Verdünnungsmittel wurde 10 mM Tris/HCl-Puffer pH 7,5, welcher zusätzlich noch 2 mM Magnesiumchlorid, 0,025 mM Zinkchlorid, 1% Ovalbumin, 1 mM Natriumdodecylsulfat und 0,02% Natriumazid enthielt, verwendet. Die Platten wurden luftdicht verschlossen bei Raumtemperatur über Nacht inkubiert. Danach wurde die Flüssigkeit abgesaugt. Die Platten wurden dreimal gespült und kurz an der Luft getrocknet. Dann wurden pro Depot 0,2 ml Puffer-Substrat-Lösung für den AP-Test eingefüllt. Nach 20 min Inkubationszeit wurde das Reaktionsgemisch aus den Depots in eine Mikroküvette übergeführt, und die Extinktion wurde im Photometer gemessen. Als Nullwert diente Puffer-Substrat-Lösung.

In den Depots, die Protease-Proteaseinhibitor-Komplexe enthielten, wurden erhöhte Extinktionswerte gefunden. Zur Auswertung wurde die gefundene Extinktion bei 405 nm in der Puffer-Substrat-Lösung nach 20 min Inkubation in den jeweiligen Depots der eingesetzten berechneten Menge an Komplex-gebundenem Thrombin gegenüber gestellt.

Die nachstehende Tabelle gibt die Endwerte der Extinktion bei 405 nm nach 20 min Reaktionszeit in Abhängigkeit von der Antigen-Menge an.

| Thrombin [ng] | OD$_{405}$/20 min |
|---|---|
| 1270 | 0,400 |
| 635 | 0,390 |
| 318 | 0,321 |
| 159 | 0,279 |
| 79,5 | 0,196 |
| 39,8 | 0,137 |
| 19,9 | 0,099 |
| 10,0 | 0,058 |
| 5,0 | 0,025 |
| 0 = Blindwert | 0 |

Die Daten zeigen, daß zwischen der eingesetzten Menge Antigen und der photometrisch gemessenen enzymatischen Indikatorreaktion ein streng korretierter Zusammenhang besteht, der eine quantitative Bestimmung von Thrombin-Antithrombin III-Komplexen ermöglicht.

### Patentansprüche

1. Verfahren zur Bestimmung von Enzymen mit Hilfe von an einen wasserunlöslichen Träger gebundenen Antikörpern gegen das zu bestimmende Enzym, dadurch gekennzeichnet, daß man Enzyme, die mit einem Inhibitor einen Enzym-Inhibitor-Komplex bilden, in Form eines solchen Komplexes mit den gebundenen Antikörpern inkubiert, die Probelösung entfernt, den ausgewaschenen, mit dem Enzym-Inhibitor-Komplex beladenen Antikörper-Träger mit einem Kupplungsprodukt, gebildet aus einem spezifisch mit dem Inhibitor reagierenden Antikörper und einer Markersubstanz, inkubiert, das ungebundene Kupplungsprodukt entfernt und die Konzentration der Markersubstanz an der festen Phase als Maß für die zu bestimmende Enzymkonzentration ermittelt.

2. Verfahren nach Anspruch 1, dadurch gekennzeichnet, daß die zu bestimmenden Enzyme Proteasen und die Inhibitoren Proteaseinhibitoren sind.

3. Verfahren nach den Ansprüchen 1 und 2, dadurch gekennzeichnet, daß das zu bestimmende Enzym Elastase aus Leukocyten und der Inhibitor α$_1$-Antitrypsin ist.

4. Mittel zur Durchführung des Verfahrens nach den Ansprüchen 1 bis 3, enthaltend mit Antikörper gegen das zu bestimmende Enzym beschichtete Reaktionsgefäße und ein Kupplungsprodukt, gebildet aus einem spezifisch mit dem Inhibitor reagierenden Antikörper und einer Markersubstanz.

5. Mittel nach Anspruch 4, enthaltend mit Antikörper gegen Proteasen beschichtete Reaktionsgefäße und ein Kupplungsprodukt, gebildet aus einem spezifisch mit dem Proteaseinhibitor reagierenden Antikörper und einer Markersubstanz.

6. Mittel nach den Ansprüchen 4 und 5, enthaltend mit Antikörper gegen Elastase aus Leukocyten beschichtete Reaktionsgefäße und ein Kupplungsprodukt, gebildet aus einem spezifisch mit $\alpha_1$-Antitrypsin reagierenden Antikörper und einem Markerenzym.

7. Verwendung des Mittels nach den Ansprüchen 4 bis 6 zur Bestimmung von Enzymen, die mit Inhibitoren einen Enzym-Inhibitor-Komplex bilden.

## Claims

1. Method for the determination of enzymes with the aid of antibodies against the enzyme to be determined, which are bonded to a water-insoluble carrier, characterised in that enzymes which, with an inhibitor, form an enzyme/inhibitor complex are incubated in form of such a complex with the bonded antibodies, the sample solution is removed, the washed antibody carrier which is charged with the enzyme/inhibitor complex is incubated with a coupling product formed from an antibody, which reacts specifically with the inhibitor, and from a marker substance, the coupling product which has not been bonded is removed and the concentration of the marker substance on the solid phase is determined, as a measure of the enzyme concentration which is to be determined.

2. Method according to Claim 1, characterised in that the enzymes which are to be determined are proteases and the inhibitors are protease inhibitors.

3. Method according to Claims 1 and 2, characterised in that the enzyme which is to be determined is elastase form leukocytes and the inhibitor is $\alpha_1$-antitrypsin.

4. Means for carrying out the method according to Claims 1 to 3, comprising test vessels, which are coated with an antibody against the enzyme which is to be determined, and a coupling product formed from an antibody, which reacts specifically with the inhibitor, and form a marker substance.

5. Means according to Claim 4, comprising test vessels, which are coated with an antibody against proteases, and a coupling product formed from an antibody, which reacts specifically with the protease inhibitor, and from a marker substance.

6. Means according to Claims 4 and 5, comprising test vessels, which are coated with an antibody against elastase from leukocytes, and a coupling product formed from an antibody, which reacts specifically with $\alpha_1$-antitrypsin, and from a marker enzyme.

7. Use of the means according to Claims 4 to 6 for the determination of enzymes which, with inhibitors, form an enzyme/inhibitor complex.

## Revendications

1. Procédé de détermination d'enzymes à l'aide d'anticorps contre l'enzyme à déterminer, ces anticorps étant fixés à un support insoluble dans l'eau, caractérisé en ce qu'on incube des enzymes formant, avec un inhibiteur, un complexe d'enzyme/inhibiteur, sous forme d'un tel complexe avec les anticorps fixés, on élimine la solution échantillon, on incube les supports d'anticorps séparés par lavage et chargés du complexe d'enzyme/inhibiteur avec un produit de couplage formé d'un anticorps réagissant spécifiquement avec l'inhibiteur, ainsi que d'une substance de marquage, on élimine le produit de couplage non fixé et on détermine la concentration de la substance de marquage dans la phase solide comme mesure de la concentration d'enzyme à déterminer.

2. Procédé suivant la revendication 1, caractérisé en ce que les enzymes à déterminer sont des protéases, tandis que les inhibiteurs sont des inhibiteurs de protéases.

3. Procédé suivant les revendications 1 et 2, caractérisé en ce que l'enzyme à déterminer est l'élastase provenant de leucocytes, tandis que l'inhibiteur est l'$\alpha_1$-antitrypsine.

4. Moyen pour la réalisation du procédé suivant les revendications 1 à 3, contenant des récipients réactionnels enduits d'anticorps contre l'enzyme à déterminer, ainsi qu'un produit de couplage formé d'un anticorps réagissant spécifiquement avec l'inhibiteur, ainsi que d'une substance de marquage.

5. Moyen suivant la revendication 4, contenant des récipients réactionnels enduits d'anticorps contre les protéases, ainsi qu'un produit de couplage formé d'un anticorps réagissant spécifiquement avec l'inhibiteur de protéase, ainsi que d'une substance de marquage.

6. Moyen suivant les revendications 4 et 5, contenant des récipients réactionnels enduits d'anticorps contre l'élastase provenant de leucocytes, ainsi qu'un produit de couplage formé d'un anticorps réagissant spécifiquement avec l'$\alpha_1$-antitrypsine et d'une enzyme de marquage.

7. Utilisation du moyen suivant les revendications 4 à 6 en vue de déterminer des enzymes formant, avec des inhibiteurs, un complexe enzyme/inhibiteur.